# EUROPEAN PATENT SPECIFICATION

(11) **EP 4 019 621 B1**
(45) Date of publication and mention of the grant of the patent: **12.03.2025**
(21) Application number: 20857194.3
(22) Date of filing: 20.07.2020
(51) Int. Cl.: G01N 21/64, G01N 21/94, G01N 15/14, G01N 15/1434, G01N 33/18, G01N 35/00

(54) **APPARATUS FOR DETECTING MICROORGANISM AND METHOD THEREFOR**
VORRICHTUNG ZUR DETEKTION VON MIKROORGANISMEN UND VERFAHREN DAFÜR
APPAREIL DE DÉTECTION DE MICRO-ORGANISME ET PROCÉDÉ ASSOCIÉ

(30) Priority: 23.08.2019 JP 2019152722
(43) Date of publication of application: 29.06.2022
(73) Proprietor: Satake Corporation, Chiyoda-ku Tokyo 101-0021 (JP)
(72) Inventor: MATSUDA Masanori, Tokyo 101-0021 (JP); FUSHIDA Shinya, Tokyo 101-0021 (JP)
(74) Representative: Haseltine Lake Kempner LLP
(86) International application number: PCT/JP2020/028165
(87) International publication number: WO 2021/039208

(56) References cited:
- WO-A1-2010/074265
- WO-A1-2019/061960
- JP-A- 2006 284 335
- JP-A- 2008 164 550
- JP-A- 2008 164 550
- JP-A- 2010 181 150
- JP-A- 2014 042 463
- US-A1- 2003 138 770
- US-A1- 2010 205 139
- US-A1- 2012 033 210
- US-A1- 2015 219 548
- US-A1- 2016 122 796

## Description

### Technical Field

The present invention relates to an apparatus for inspecting ballast water, and in particular, to an inspection apparatus that is suitable for detecting live microorganisms, such as plankton, contained in ballast water, for example.

### Background Art

A vessel not loaded with cargo sails while being loaded with ballast water to make it stable, and discharges the ballast water in a sea area where cargo is loaded onto the vessel.

Ballast water is usually discharged in a sea area different from a sea area where the ballast water has been loaded. Therefore, microorganisms, such as plankton and bacteria, contained in the ballast water are carried to a sea area other than the native habitat of the microorganisms, and thus may damage the ecosystem.

To address such a problem, an international rule about the regulation of ballast water has been established. Specifically, the "International Convention for the Control and Management of Ships' Ballast Water and Sediments (Ballast Water Management Convention)" has been enacted and has entered into force.

In "Guidelines for ballast water sampling (G2)" related to the aforementioned Ballast Water Management Convention, "Ballast Water Performance Standard (D-2)" specifies the allowable limits for the number of live microorganisms contained in ballast water discharged from vessels based on the minimum size (i.e., minimum diameter) of the microorganisms. For example, the standard specifies that vessels shall discharge less than 10 organisms/m³ regarding organisms with a minimum size (i.e., minimum diameter) of 50 µm or greater (hereinafter referred to as "L-size organisms") and less than 10 organisms/ml regarding microorganisms with a minimum size (i.e., minimum diameter) of 10 µm or greater and less than 50 µm (hereinafter referred to as "S-size organisms").

As a method for confirming if the aforementioned discharge standard is met during the discharge of the ballast water, there is known a method of passing sea water, which has been pumped with a conveying pump, through a flow cell and performing measurement based on an image (for example, Patent Literature 1). As another means, there is also known a method of passing sea water, which has been pumped with a conveying pump, through a filter unit including filters with different apertures, and causing microorganisms on the filters to emit light, and then counting the number of the microorganisms (for example, Patent Literature 2). As further another means, there is also known a microorganism inspection apparatus for inspecting the number of microorganisms using a batch-type measurement cell (for example, Patent Literature 3).

A microorganism inspection apparatus described in Patent Literature 1 above includes a staining unit for staining organisms having live cells existing in a flowing liquid specimen, a concentration unit for increasing the concentration of the organisms in the flow of a stained specimen, an individual measuring unit for acquiring image information on the individuals containing the organisms in the concentrated specimen, and a control unit for measuring the organisms based on the image information on the individuals output from the individual measuring unit.

Accordingly, it is possible to perform flow cytometry inclusive of the step of staining organisms in a liquid specimen, the step of increasing the concentration of the organisms in the liquid, and the step of acquiring information on the organisms in the liquid, for example. Therefore, in comparison with a method of performing each step through a batch process, it is possible to significantly reduce the standby time for part of a specimen, which has undergone one step, to proceed to a next step, or set the standby time to zero, and thus acquire stable information about whether the organisms are alive or dead in the sense that degradation of the organisms in the stained state during the standby time can be prevented.

In addition, a microorganism inspection apparatus described in Patent Literature 2 above includes a step of passing sea water through a filter unit in which three types of filters with different apertures are arranged in series, a step of causing live microorganisms trapped by the filters to exhibit a color, emit light, or emit fluorescence, and a step of detecting one of the exhibited color, emitted light, and emitted fluorescence, and performing image analysis to count the number of microorganisms in the ballast water or the sea water.

Accordingly, it is possible to trap microorganisms of each size in stages, and thus quickly measure if the standard regulating the allowable residual microorganisms of each size is met.

Further, a microorganism inspection apparatus described in Patent Literature 3 above is adapted to add a sample and a fluorescent staining reagent into a batch-type sample container formed of a light-transmitting material, and stir and mix the sample solution, and then irradiate the sample solution with a light beam from a light source so as to calculate the number of microorganisms contained in a sample of the sample solution.

A microorganism inspection apparatus of Patent Literature 4 has a sample container for holding a sample and a fluorescent staining reagent, a stirring and mixing means, an excitation light source and a light receiving means.

Accordingly, it is possible to easily detect and measure the number of microorganisms in ballast water in a short time with high accuracy.

### Citation List

### Patent Literature

Patent Literature 1: Japanese Patent Laid-Open No. 2009-85898
Patent Literature 2: Japanese Patent Laid-Open No. 2007-135582
Patent Literature 3: Japanese Patent Laid-Open No. 2014-42463
Patent Literature 4: US Patent Application No. 2015/219548 A1

### Summary of Invention

### Technical Problem

The microorganism inspection apparatus described in Patent Literature 1 is adapted to sequentially pass sea water, which has been pumped with a conveying pump, through each step. Thus, the apparatus is large and the production cost may become high. In addition, although the sea water is sequentially passed through each step to shorten the standby time, it may take at least several hours to complete the measurement.

The microorganism inspection apparatus described in Patent Literature 2 is also adapted to sequentially pass sea water, which has been pumped with a conveying pump, through each step as with the technique described in Patent Literature 1. Thus, the apparatus is large and the production cost may become high.

In the microorganism inspection apparatus described in Patent Literature 3, a light-receiving unit is provided such that its light-receiving surface is arranged at an angle perpendicular to an excitation light beam. Thus, a number of optical components, such as a means for converting a light beam into a collimated light beam, a relay lens, a slit, and a condensing lens, are used. In addition, since a light source unit is provided on the lateral face side of the sample container, and the light-receiving unit is provided on the front face side or the rear face side of the sample container, the area for disposing the light source unit and the light-receiving unit as seen in plan view is large, which may result in a large body of the inspection apparatus.

It is a technical object of the present invention to provide a microorganism inspection apparatus with a reduced number of components and a reduced production cost, and also with a reduced size, and in which irradiation unevenness of an excitation light beam from a light source unit can be suppressed and thus inspection accuracy can be improved.

### Solution to Problem

To solve the aforementioned problems, the present invention provides as a technical means a microorganism inspection apparatus for measuring the number of microorganisms in a sample as set out in the appended set of claims.

In addition, the excitation light source may be arranged above or below the sample container. Further, a reflective member may be provided in the sample container, the excitation light source may be arranged on a lateral side of the sample container, and an excitation light beam emitted from the excitation light source may irradiate the irradiation plane in the vertical direction by being reflected by the reflective member.

In addition, the microorganism inspection apparatus may further include an optical guide bar between the excitation light source and the irradiation plane.

Further, the microorganism inspection apparatus may further include a shielding plate on a side opposite to the excitation light source across the irradiation plane.

Accordingly, in comparison with an apparatus that performs measurement by leaving a sample solution to stand without stirring it, it is possible to allow microorganisms to emit bright light in an extremely short time and thus easily measure the number of microorganisms in ballast water in a short time. In addition, since the apparatus of the present invention uses a batch-type sample container, the size of the apparatus can be reduced, and the production cost can also be reduced. Further, since the number of optical members and the like can be reduced in comparison with that of the conventional art, the size of the apparatus can be further reduced, and the production cost can be further reduced. Furthermore, since excitation light beam irradiation is performed in a direction perpendicular to the water flow of the sample solution, the influence of the water flow can be reduced, and detection accuracy can be improved with suppressed irradiation unevenness.

In addition, since the excitation light source is arranged above or below the sample container, it is possible to reduce the number of members arranged around the sample container and the measurement unit as seen in plan view, and thus reduce the size of the apparatus and reduce the production cost.

Further, since the excitation light source is arranged on the lateral side of the sample container, the flexibility of the arrangement of the excitation light source can be increased. Furthermore, since excitation light beam irradiation is performed in a direction perpendicular to the water flow of the sample solution, the influence of the water flow can be reduced, and detection accuracy can be improved with suppressed irradiation unevenness.

In addition, since the optical guide bar is provided between the excitation light source and the irradiation plane, it is possible to allow an excitation light beam from the excitation light source to reliably irradiate the irradiation plane, and thus further improve detection accuracy.

Further, even when a lid and the like are not provided, it is possible to perform measurement without the operator feeling dazzling. Furthermore, the measurement range of microorganisms, including its vicinity, can be reliably irradiated with an excitation light beam.

### Advantageous Effects of Invention

According to the present invention, it is possible to reduce the number of components and reduce the production cost, and also reduce the size of an inspection apparatus, and further suppress irradiation unevenness of an excitation light beam from a light source unit, and thus improve inspection accuracy.

### Brief Description of Drawings

[Figure 1] Figure 1 is a perspective view illustrating the entire microorganism inspection apparatus according to an embodiment of the present invention.
[Figure 2] Figure 2 is a diagram illustrating the electrical control configuration of the inspection apparatus according to the present embodiment.
[Figure 3] Figure 3 is a side view of a measurement unit according to the present embodiment.
[Figure 4] Figure 4 is a view illustrating exemplary shapes of a diaphragm.
[Figure 5] Figure 5 is a chart illustrating a measurement flow according to the present embodiment.
[Figure 6] Figure 6 is a side view of a measurement unit according to another embodiment of the present invention.
[Figure 7] Figure 7 is a side view of a measurement unit according to further another embodiment of the present invention.
[Figure 8] Figure 8 is a side view of a measurement unit according to still another embodiment of the present invention.
[Figure 9] Figure 9 is a side view of a measurement unit according to yet another embodiment of the present invention.
[Figure 10] Figure 10 is a side view of a measurement unit according to yet another embodiment of the present invention.
[Figure 11] Figure 11 is a side view of a measurement unit according to yet another embodiment of the present invention.
[Figure 12] Figure 12 is a schematic planar cross-sectional view of a measurement unit of conventional art.
[Figure 13A] Figure 13A is a schematic cross-sectional view illustrating a flat plate with a predetermined thickness having a thread cutting hole formed therein as an embodiment of a means for converting a light beam into a collimated light beam.
[Figure 13B] Figure 13B is a schematic cross-sectional view illustrating an LED light source with a front face provided with a convex lens as an embodiment of a means for converting a light beam into a collimated light beam.
[Figure 14] Figure 14 is a view illustrating a detection method performed with a conventional inspection apparatus.

### Description of Embodiments

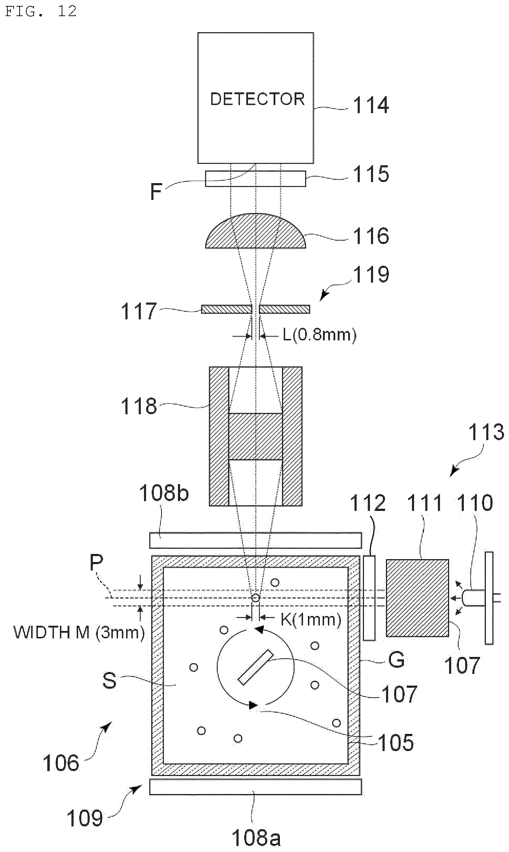

To describe the characteristics of the invention of the present application, a summary of conventional art described in Patent Literature 3 will be described first. Figure 12 is a schematic planar cross-sectional view of a measurement unit of the conventional art.

An inspection apparatus of the conventional art includes a batch-type sample container 105 formed of a light-transmitting, transparent material (for example, glass, quartz, or acrylic resin), and a measurement unit 106 that optically counts the number of microorganisms in the sample container 105. The sample container 105 includes a rotor 107 to stir a sample solution S in the sample container 105. The rotor 107 is configured to be rotationally driven by a stirrer provided in a body (not illustrated).

The inspection apparatus illustrated in Figure 12 is formed such that it has a width of 300 mm, a depth of 300 mm, a height of 100 mm, and a weight in the range of about 2 to 4 kg. Therefore, the inspection apparatus can be carried around while being put in a handheld trunk or a backpack (not illustrated), for example, and thus can perform measurement on a vessel or outside.

In addition, the batch-type sample container 105 formed of a light-transmitting, transparent material is formed in the shape of a prism having a bottom face with a size of 50 mm × 50 mm and a height of 60 mm, and has an internal volume set to 100 ml (milliliter) when the water level in the container is 40 mm. The shape of the sample container 105 is not limited to such a prism shape, and may be a cylindrical shape or a cubic shape as long as an internal volume of about 100 ml (milliliter) can be secured.

The measurement unit 106 includes a sample container housing unit 109 that houses and holds the sample container 105, a light source unit 113 that emits an excitation light beam toward the sample container 105, and a light-receiving unit 119 for observing microorganisms stained by a luminescence reagent and floating in the sample container 105 with the excitation light beam emitted from the light source unit 113. The light-receiving unit 119 is electrically connected to a CPU (not illustrated) that counts the number of microorganisms in the sample solution S and performs an information processing operation or a statistical processing operation on the measurement results, for example.

The sample container housing unit 109 is formed with holding plates 108a and 108b surrounding at least two faces of the sample container 105, and houses and holds the sample container 105 so as not to block a light beam emitted from the light source unit 113.

In addition, as illustrated in Figure 12, the light source unit 113 is arranged so as to allow an excitation light beam to become incident on an irradiation plane G of the sample container 105 in the direction of the normal P to the irradiation plane G. The light source unit 113 includes an LED light source 110 arranged in the vicinity of the sample container housing unit 109, a means 111 for converting a light beam into a collimated light beam, arranged on the front face side of the LED light source 110 and adapted to convert a diffused light beam into a collimated light beam, and an excitation filter 112 that irradiates the sample container 105 with an excitation light beam of a slit-collimated light beam.

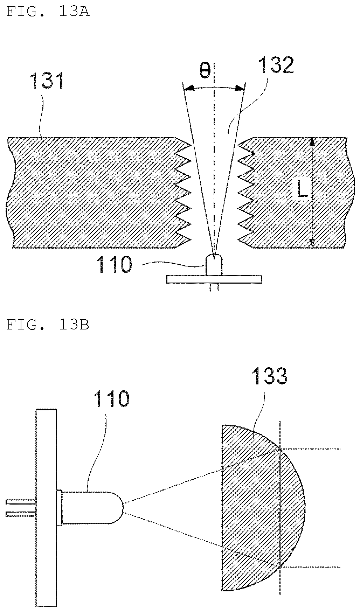

Figures 13 are schematic cross-sectional views each illustrating an embodiment of the means 111 for converting a light beam into a collimated light beam. In the example illustrated in Figure 13A, a flat plate 131 with a predetermined thickness, which has a thread cutting hole 132 with a predetermined diameter drilled therein, is formed as the means 111 for converting a light beam into a collimated light beam, and the thickness L of the flat plate 131 and the diameter of the thread cutting hole are appropriately set in accordance with the optical path length. This allows a scattered light beam with an incidence angle θ emitted from the LED light source 110 to be converted into a collimated light beam while passing through the thread cutting hole 132. In the example illustrated in Figure 13A, the optimum conditions for θ and L are determined through a test of the SN ratio, and for example, provided that the thread cutting hole 132 has a size of M3 (the outside diameter of the threaded hole) × 0.5 (pitch), the optimum conditions are θ of 9.5° and L of 15 mm.

The means 111 for converting a light beam into a collimated light beam illustrated in Figure 13B is the LED light source 110 with a front face provided with a convex lens 133. A scattered light beam emitted from the LED light source 110 is converted into a collimated light beam when output to the outside through the convex lens 133.

The light-receiving unit 119 is provided such that its light-receiving surface F is arranged at an angle perpendicular to an excitation light beam in the direction of the normal P from the light source unit 113. The light-receiving unit 119 includes a detector 114 arranged and configured to receive fluorescence along an optical axis perpendicular to an excitation light beam emitted from the LED light source 110 toward the sample container 105, a light-receiving filter 115 arranged on the front face side of the detector 114, a condensing lens 116 arranged on the front face side of the light-receiving filter 115, a slit 117 arranged on the front face side of the condensing lens 116, and a relay lens 118 arranged in the gap between the slit 117 and the sample container 105 and adapted to excite a fluorescent material contained in microorganisms and collect the resulting fluorescence emission to form an image.

In such an inspection apparatus, since the rotor 107 rotates on the bottom face of the sample container 105 as illustrated in Figure 12, the sample solution S basically rotates in the direction along the plane of the bottom face. Usually, the measurement range is set vertically long in the vertical direction so as to allow for efficient detection of the number of microorganisms based on the direction of rotation of the sample solution S.

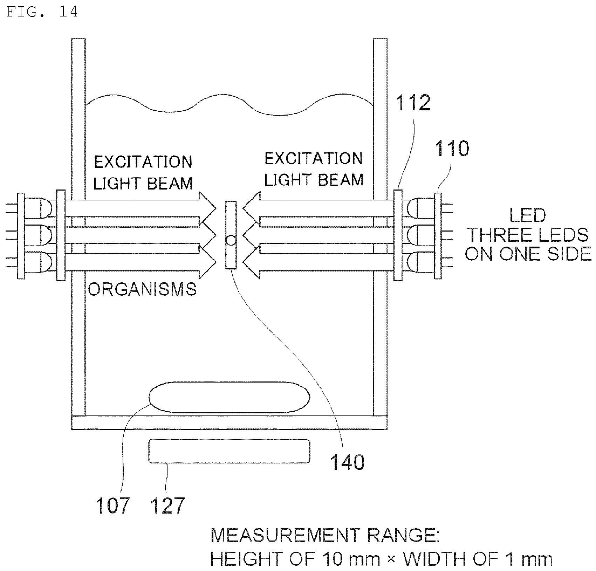

Figure 14 is a view illustrating a detection method performed with the conventional inspection apparatus. A measurement range 140 is set vertically long to specifically allow for the detection of fluorescence emitted from organisms and passing through a region with a height of 10 mm and a width of 1 mm. Due to such a configuration, LED light sources 110 are arranged at three points in the conventional inspection apparatus to cover the entire measurement range 140 with a height of 10 mm. To detect a light beam from an intended observation point among light beams from the three points, the light-receiving unit includes optical components, such as the condensing lens 116, the slit 117, and the relay lens 118. Therefore, in the conventional art, it is necessary to dispose the light source unit 113 and a number of optical components as the light-receiving unit 119 around the sample container 105, which may result in an increased overall size of the inspection apparatus.

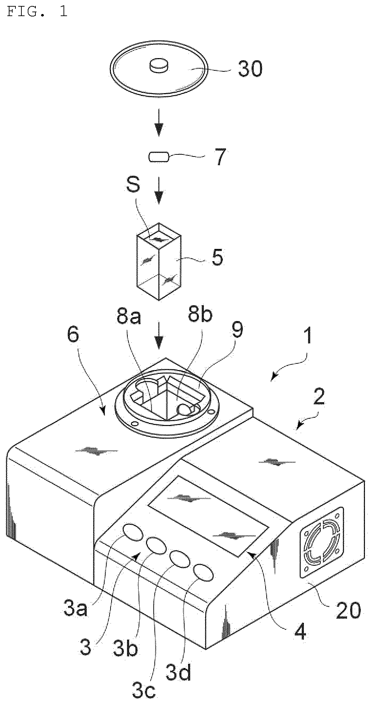

In view of the foregoing, an embodiment of the present invention will be described hereinafter. Figure 1 is a perspective view illustrating the entire microorganism inspection apparatus according to an embodiment of the present invention.

An inspection apparatus 1 of the present embodiment includes a body unit 2 that incorporates a control mechanism, such as a CPU board, and performs an operation of processing measurement results, for example, an operation unit 3 that is arranged next to the body unit 2 and includes operation buttons, for example, and a display unit 4 for displaying the measurement results, for example. The display unit 4 includes a liquid crystal panel, for example. The operation unit 3 includes a power button 3a, a measurement start button 3b, an external output button 3c, and a setting button 3d. Pressing the power button 3a can control the on/off switching, and pressing the measurement start button 3b can start measurement. In addition, pressing the external output button 3c can transfer data to an external printer or personal computer, and pressing the setting button 3d can switch the type of measurement, such as the setting of the size of microorganisms as a measurement target, change the setting of a threshold, and change the setting of the measurement time.

The body unit 2 includes a measurement unit 6. The measurement unit 6 houses a batch-type sample container 5 and optically counts the number of microorganisms in a sample solution S in the sample container 5. The sample container 5 is formed of a transparent material, such as glass, quartz, or acrylic resin, for example, and thus is configured to transmit light. The sample container 5 houses a rotor 7, and the rotor 7 stirs the sample solution S. The rotor 7 is housed in the sample container 5 together with the sample solution S and a luminescence reagent, and is closed with a lid 30. In addition, the rotor 7 is configured to be, when the sample container 5 is housed in the measurement unit 6, rotationally driven by a stirrer 27 incorporated in the measurement unit 6.

The inspection apparatus 1 illustrated in Figure 1 is dimensioned such that it can be carried around while being put in a handheld trunk, for example. Therefore, it is possible to perform measurement on a vessel or outside by carrying around the inspection apparatus.

The sample container 5 is formed in the shape of a prism having a bottom face with a size of 50.5 mm × 51.5 mm and a height of 67 mm, and has a volume set to about 100 ml when the water level in the container is 43 mm. Although the sample container 5 is formed in the shape of a prism in the present embodiment, the sample container 5 may have other shapes, such as a cylindrical shape. In such a case, however, the sample container 5 is desirably dimensioned to be able to secure a volume of about 100 ml to facilitate measurement.

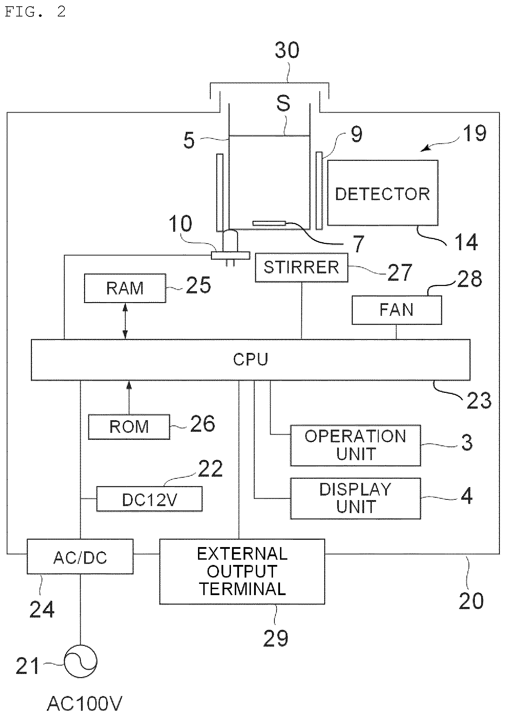

Further, the electrical control configuration of the inspection apparatus 1 of the present embodiment will be described based on Figure 2. A CPU board 23, which is supplied with power from an AC power supply 21 or a secondary battery 22, is arranged in the center of a housing 20 forming the body unit 2. The CPU board 23 analyzes an output signal obtained by converting a light beam into electricity with a detector 14, determines if the luminance is greater than or equal to a given luminance level, counts pulses of a given luminance signal, and controls on/off of an LED light source 10, for example. An AC/DC converter 24 is arranged between the AC power supply 21 and the CPU board 23.

The CPU board 23 has electrically connected thereto the detector 14, the LED light source 10, a RAM 25 as a read/write memory portion, and a ROM 26 as a read-only memory portion.

Besides, the CPU board 23 has connected thereto the stirrer 27, which rotates the rotor 7 with a magnetic force, the display unit 4 formed with a liquid crystal panel, for example, a cooling fan 28 for control devices, such as the CPU board 23, and an external output terminal 29, such as RS-232C.

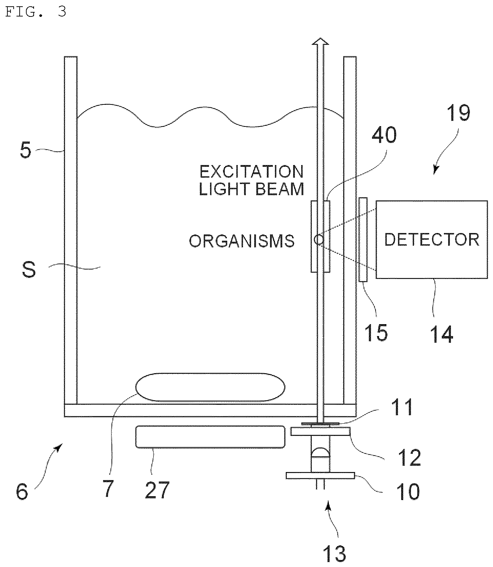

Next, the configuration of the measurement unit 6 of the present embodiment will be described based on Figure 3. Figure 3 is a side view of the measurement unit 6 of the present embodiment. The sample solution S and the rotor 7 are put in the sample container 5, and the rotor 7 can be rotated by the stirrer 27 provided in the measurement unit 6. A light source unit 13 is provided on the bottom face side of the sample container 5, and an excitation filter 12 is provided on the front face side of the LED light source 10, and further, a diaphragm 11 is provided on the front face side of the excitation filter 12. As the excitation filter 12, a bandpass filter can be used. The shape of the diaphragm will be described later. The light-receiving unit 19 is provided on the lateral face side of the sample container 5, and includes the detector 14 and a light-receiving filter 15. As the detector 14, it is possible to use a photomultiplier, for example, that is arranged and configured to receive fluorescence emitted by microorganisms that have been irradiated with a light beam emitted from the light source unit 13 toward the sample solution S in the sample container 5. As the color of the LED light source 10, blue is preferably used.

The rotor 7 is arranged on the bottom face of the sample container 5. Thus, when the rotor 7 is rotated by the stirrer 27, the flow of water is stirred mainly in the horizontal direction along the bottom face. Therefore, although excitation light beam irradiation is performed in a direction parallel with the flow of water in the conventional art, in the present embodiment, excitation light beam irradiation is performed in a direction perpendicular to the flow of water. Therefore, the influence of the flow of water is reduced, and detection of the number of microorganisms can be performed with higher accuracy and with suppressed irradiation unevenness.

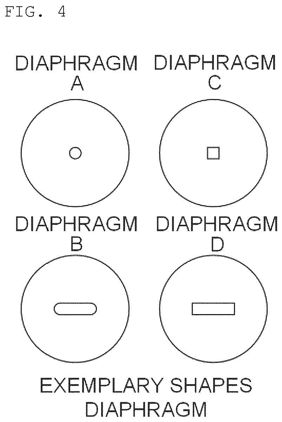

Figure 4 illustrates exemplary shapes of the diaphragm 11. A diaphragm A has a circular shape, a diaphragm B has a shape with arc-like portions, such as an elliptical shape, an elongated circular shape, or a rounded rectangular shape, a diaphragm C has a square shape, and a diaphragm D has a rectangular shape. Such diaphragms are selectively used according to the intended use.

An excitation light beam excited by the LED light source 10 passes through the excitation filter 12 so that unwanted wavelengths are filtered out, and then, the beam shape is adjusted by the diaphragm 11. Accordingly, it is possible to reduce variation in the amount of fluorescence emitted by microorganisms depending on the portion through which an excitation light beam passes.

In the present embodiment, the number of optical members on the side of the light source unit 13 and the side of the light-receiving unit 19 can be reduced in comparison with that of the conventional art, and thus, the optical configuration can be simplified. Specifically, the number of the LED light sources 10 can be reduced from 6 to 1, and the number of the excitation filters 12 can be reduced from 2 to 1. The diameter of the LED light source 10 can also be reduced to half, specifically, from 25 mm to 12.5 mm. In addition, the number of pieces of cover glass provided on the excitation filter 12 can also be reduced from 2 to 1, and the size of the cover glass can also be reduced.

Regarding the light source unit 13, although the LED light source 10 is used as a light source, it is possible to use not only the LED light source 10 but also a collimated-light-beam LED light source capable of emitting a collimated light beam, a laser light source, or a light bulb as long as a fluorescent material contained in microorganisms can be excited.

Regarding the optical components on the side of the light-receiving unit 19, there is no need to use a relay lens, a slit, or a cylindrical lens provided in the conventional art. In addition, regarding the light-receiving filter 15 and the detector 14, a single light-receiving filter 15 and a single detector 14 may be provided.

In this manner, reducing the number of optical components on the side of the light source unit 13 and the side of the light-receiving unit 19 to simplify the configuration can reduce the overall size of the apparatus.

Although the sample container 5 in the present embodiment is formed in the shape of a prism, the sample container 5 may be formed in a cylindrical shape. Further, the sample container 5 may also be formed in the shape of a polygonal prism, such as a triangular prism, a pentagonal prism, or a hexagonal prism.

Although an example in which a photomultiplier (PMT) is used as a light-receiving sensor of the light-receiving unit 19 has been illustrated, the present invention is not limited thereto, and it is possible to use various photodetectors, such as a silicon photodiode (SiPD) and an avalanche photodiode (APD), that can detect light emission of a fluorescent material contained in microorganisms as with a photomultiplier (PMT).

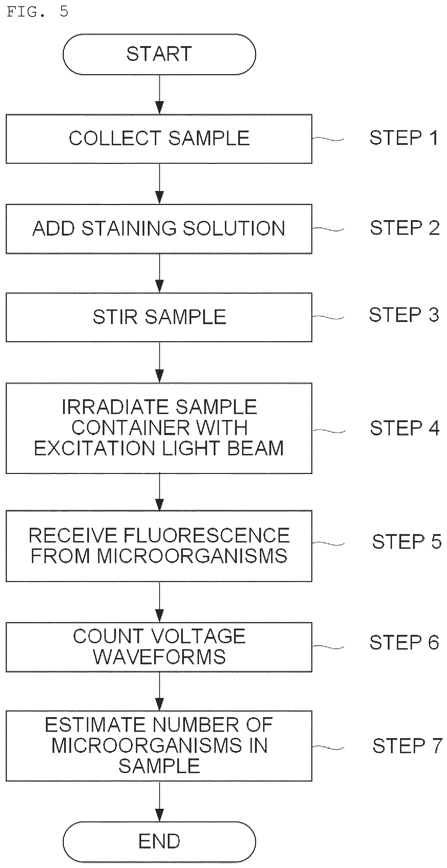

Figure 5 is a flowchart illustrating a measurement flow. A measurement flow of each step will be described based on Figure 5.
· (Step 1) An operator collects 100 ml (milliliter) of a sample from ballast water at a temperature of about 20°C, using a pipette, for example, and then puts the sample into the sample container 5.
· (Step 2) A fluorescent staining reagent is added into the sample container 5. As the fluorescent staining reagent, it is possible to use commonly known calcein AM (Calcein-AM manufactured by Promocell GMBH in Germany) or FDA (Fluorescein Diacetate manufactured by Tokyo Chemical Industry Co., Ltd.), for example. Calcein AM tends to stain phytoplankton more easily, while FDA tends to stain zooplancton more easily. Therefore, when staining with a staining reagent is performed with a reagent containing a mixture of calcein AM and FDA, it is possible to shorten the reagent staining time and specifically reduce the time required for staining to half that of the conventional art.
· (Step 3) The operator puts the rotor 7 into the sample container 5 and places the sample container 5 in the measurement unit 6 of the inspection apparatus 1, and then puts the lid 30 of the measurement unit 6, thereby completing the preparation for measurement. Then, the operator presses the power button 3a to cause the rotor 7 to rotate with the drive of the magnetic stirrer 27 incorporated in the measurement unit 6, and thus stir the sample solution S.
· (Step 4) The operator presses the measurement start button 3b of the operation unit. Then, after a predetermined time has elapsed, the LED light source 10 is lighted so that the sample container 5 is irradiated with a light beam transmitted through the bandpass filter 12 for an excitation light beam. At this time, for example, the sample container 5 is irradiated with a light beam with a wavelength of 450 nm to 490 nm as the wavelength characteristics so that a specimen (i.e., microorganisms) in the sample container 5 emits fluorescence.
· (Step 5) The detector 14 of the light-receiving unit 19 detects the fluorescence.
· (Step 6) The detector 14 detects the emission of fluorescence by converting the light energy into electrical energy utilizing the photoelectric effect, and also with a current amplification function added thereto. The detected electric signal is sent to the CPU board 23 so that received-light waveforms that are greater than or equal to a given threshold are counted.
· (Step 7) Further, the CPU board 23 estimates the number of microorganisms existing in 100 ml (milliliter) of water in the sample container 5 from the counted value of the received-light waveforms, and then displays on the display unit 4 information about whether the estimated number of microorganisms satisfies the effluent standard.

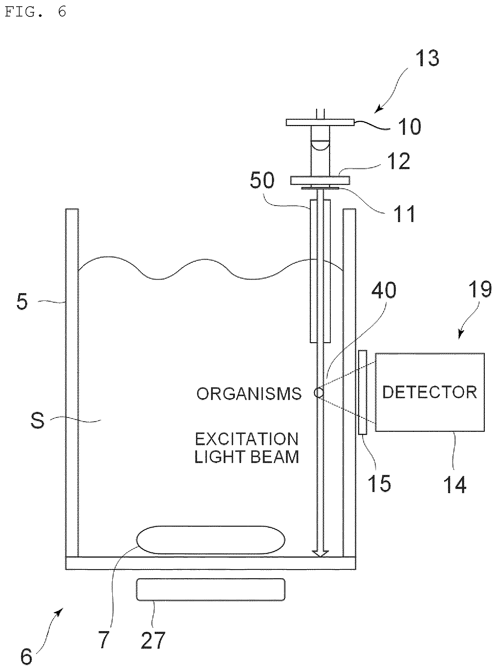

The configuration of the measurement unit 6 according to another embodiment will be described based on Figure 6. The present embodiment differs from the previous embodiment in that the light source unit 13 is arranged above the sample container 5. When light beam irradiation is performed from above as in the present embodiment, the light beam may not travel straight and may not reach the inside of the water due to ruffle generated at the boundary portion between the air and the water surface or due to the difference in refractive index between the air and the water if no measure is taken. Thus, an optical guide bar 50 is provided ahead of the diaphragm 11 of the light source unit 13, and the optical guide bar 50 is arranged in a range including the air and the water. In the present embodiment, the lid 30 is preferably provided with a hole for passing the optical guide bar 50. A light beam emitted from the LED light source 10 and transmitted through the excitation filter 12 and the diaphragm 11 passes through the optical guide bar 50, and thus is guided to the vicinity of the measurement unit. The optical guide bar 50 is formed of transparent resin, such as acrylic resin, and guides a light beam utilizing internal reflection. Accordingly, the influence between the air and the water can be reduced, and a light beam from the light source unit 13 can be guided to the vicinity of the measurement unit for microorganisms. The other configurations of the light source unit 13 and the light-receiving unit 19 are similar to those of the previous embodiment. Thus, the description thereof is omitted herein.

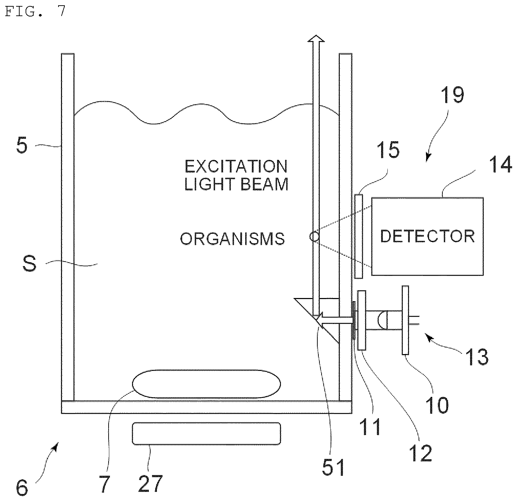

The configuration of the measurement unit 6 according to further another embodiment will be described based on Figure 7. The present embodiment differs from the previous embodiments in that the light source unit 13 is arranged on the same side as the light-receiving unit 19 that is arranged on the lateral side of the sample container 5. In the present embodiment, a mirror 51 is arranged in the sample solution S to allow microorganisms to be irradiated with a light beam from below. Specifically, the direction of a light beam from the light source unit 13 is switched to the vertical direction so that the light beam irradiates the microorganisms. The light-receiving unit 19 is arranged so as to receive a light beam which the microorganisms emit as fluorescence, which has become incident on the microorganisms in the vertical direction, at an angle perpendicular thereto. The other configurations of the light source unit 13 and the light-receiving unit 19 are similar to those of the previous embodiments. Thus, the description thereof is omitted herein. Although the mirror 51 is used to switch the direction of a light beam in the present embodiment, it is also possible to use other elements, such as a prism 52, to switch the direction of a light beam. In the present embodiment, since the light source unit 13 and the light-receiving unit 19 are arranged on the same lateral side, the size of the inspection apparatus can be reduced.

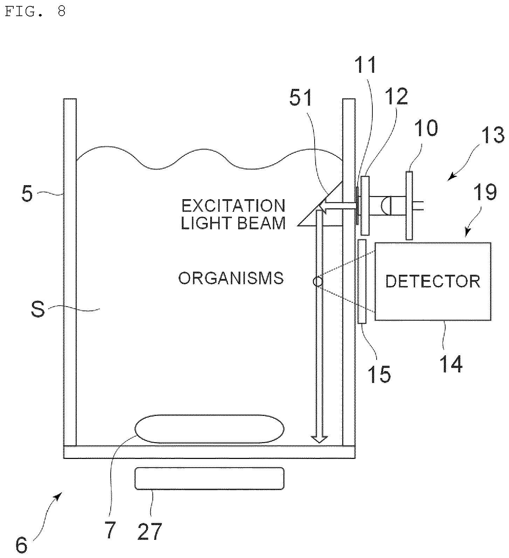

The configuration of the measurement unit 6 according to still another embodiment will be described based on Figure 8. In the present embodiment, the light source unit 13 is arranged on the same side as the light-receiving unit 19 that is arranged on the lateral side of the sample container 5 as in the previous embodiment. In the present embodiment, the light source unit 13 is arranged at a position above the light-receiving unit 19, and a mirror 51 is arranged in the sample solution S so as to allow microorganisms to be irradiated with a light beam from above. Specifically, the direction of a light beam from the light source unit 13 is switched to the vertical direction so that the light beam irradiates the microorganisms. The light-receiving unit 19 is arranged so as to receive a light beam which the microorganisms emit as fluorescence, which has become incident on the microorganisms in the vertical direction, at an angle perpendicular thereto. The other configurations of the light source unit 13 and the light-receiving unit 19 are similar to those of the previous embodiments. Thus, the description thereof is omitted herein. Although the mirror 51 is used to switch the direction of a light beam in the present embodiment, it is also possible to use other elements, such as a prism 52, to switch the direction of a light beam. In the present embodiment, the light source unit 13 and the light-receiving unit 19 are also arranged on the same lateral side. Thus, the size of the inspection apparatus can be reduced.

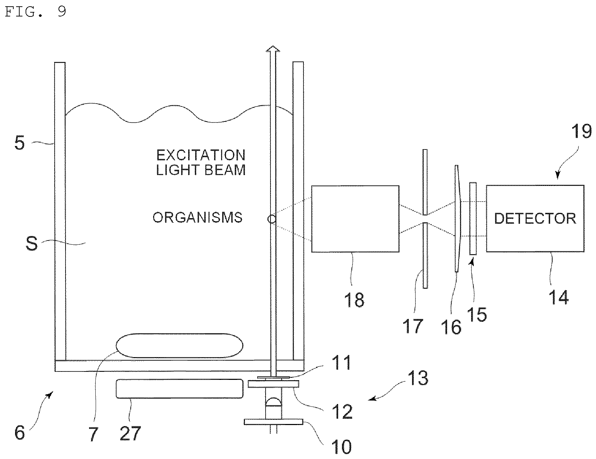

Figure 9 illustrates the configuration of the measurement unit 6 according to yet another embodiment. The present embodiment differs from the other embodiments in the configuration of the light-receiving unit 19. The light-receiving unit 19 according to the present embodiment is provided on the lateral face side of the sample container 5, and includes the detector 14 and the light-receiving filter 15. A condensing lens 16 is arranged on the front face side of the light-receiving filter 15, and a slit 17 is arranged on the front face side of the lens 16. In addition, a relay lens 18 is arranged between the slit 17 and the sample container 5. The relay lens 18 has functions of exciting a fluorescent material contained in microorganisms, and collecting the resulting fluorescence emission to form an image.

The slit 17 is used to narrow the observation plane in a slit form. Using the slit 17 can narrow the light-receiving area of the light-receiving surface and can also narrow the area of the background fluorescence emission that would become noise. This improves the ratio of a signal of the fluorescence emission of microorganisms to the background fluorescence emission, and thus improves detection accuracy for the fluorescence emission of microorganisms.

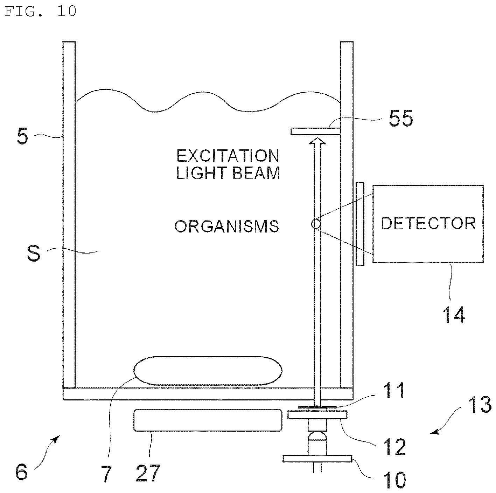

The configuration of the measurement unit 6 according to yet another embodiment will be described based on Figure 10. In the present embodiment, the light source unit 13 is arranged below the sample container 5 so as to emit an excitation light beam vertically upward, but the present embodiment differs from the previous embodiment in that a light-shielding plate 55 is arranged in the vicinity of the water surface. Such a configuration allows a light beam, which has irradiated microorganisms and passed therethrough in the upward direction, to be shielded by the light-shielding plate 55. Thus, even when the lid 30 is not provided, it is possible to perform measurement without the operator feeling dazzling. The other configurations of the light source unit 13 and the light-receiving unit 19 are similar to those of the previous embodiments. Thus, the description thereof is omitted herein.

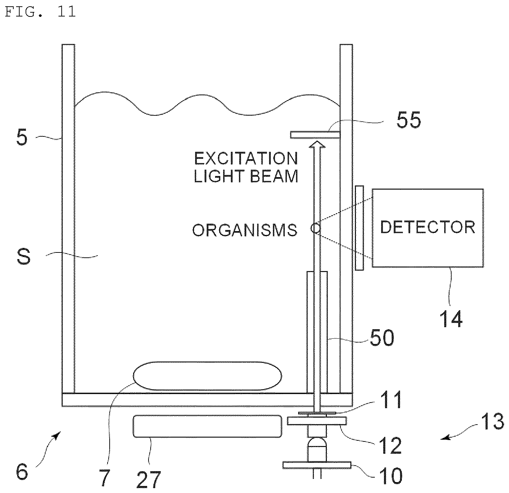

The configuration of the measurement unit 6 according to yet another embodiment will be described based on Figure 11. The present embodiment differs from the previous embodiment in that the optical guide bar 50 is provided for excitation light beam irradiation in addition to the light-shielding plate 55 provided as in the previous embodiment so that an excitation light beam can be reliably transmitted from the bottom face of the sample container 5 to the vicinity of the measurement range of microorganisms. Such a configuration allows a light beam, which has irradiated the microorganisms and passed therethrough in the upward direction, to be shielded by the light-shielding plate 55. Thus, even when the lid 30 is not provided, it is possible to perform measurement without the operator feeling dazzling. Further, the measurement range of microorganisms, including its vicinity, can be reliably irradiated with an excitation light beam. The other configurations of the light source unit 13 and the light-receiving unit 19 are similar to those of the previous embodiments. Thus, the description thereof is omitted herein.

Although the aforementioned embodiments have illustrated examples in which a single LED light source is provided, it is also possible to arrange a plurality of LED light sources, such as three LED light sources. In such a case, if the LED light sources are arranged on the bottom face side of the sample container 5, for example, the LED light sources are preferably arranged in the horizontal direction along the direction of the flow of the sample solution S based on the rotor 7. The rotor 7 is arranged on the bottom face and excitation light beam irradiation is performed in the vertical direction with respect to the rotor 7.

### Reference Signs List

- 1: Inspection apparatus
- 2: Body unit
- 3: Operation unit
- 4: Display unit
- 5: Sample container
- 6: Measurement unit
- 7: Rotor
- 8: Holding plate
- 9: Sample container housing unit
- 10: LED light source
- 11: Diaphragm
- 12: Excitation filter
- 13: Light source unit
- 14: Detector
- 15: Light-receiving filter
- 16: Condensing lens
- 17: Slit
- 18: Relay lens
- 19: Light-receiving unit
- 20: Housing
- 21: AC power supply
- 22: Secondary battery
- 23: CPU board
- 24: AC/DC converter
- 25: RAM
- 26: ROM
- 27: Stirrer
- 28: Fan
- 29: External output terminal
- 30: Lid
- 50: Optical guide bar
- 51: Mirror
- 52: Prism
- 55: Light-shielding plate

## Claims

1. A microorganism inspection apparatus (1) for measuring the number of microorganisms in a sample solution, comprising:
a batch-type sample container formed of a light-transmitting material;
stirring/mixing means (7, 27) for stirring/mixing a sample and a fluorescent staining reagent in the batch-type sample container (5) to form a sample solution;
an excitation light source (13) including a light source (10) that irradiates a horizontal irradiation plane of the sample container (5) with an excitation light beam in a vertical direction while the sample solution is stirred by the stirring/mixing means;
light-receiving means (14) arranged on a lateral face side of the sample container (5), the light-receiving means being configured to detect a fluorescence beam emitted in response to the excitation light beam from the excitation light source (13); and
control means (23) for converting the beam detected by the light-receiving means (14) into an electric signal to detect the number of light emissions, and calculating the number of microorganisms contained in the sample in the sample container (5) from the number of light emissions, wherein
the stirring/mixing means (7, 27) and the excitation light source (13) are arranged such that a direction of the flow of the sample solution due to agitation by the stirring/mixing means and a direction of the irradiation of the excitation light beam from the excitation light source are perpendicular to each other.

2. The microorganism inspection apparatus (1) according to claim 1, wherein the excitation light source (13) is arranged above or below the sample container (5).

3. The microorganism inspection apparatus (1) according to claim 1,
wherein:
a reflective member is provided in the sample container,
the excitation light source (13) is arranged on a lateral side of the sample container (5), and
an excitation light beam emitted from the excitation light source irradiates the irradiation plane in the vertical direction by being reflected by the reflective member.

4. The microorganism inspection apparatus (1) according to any one of claims 1 to 3, further comprising an optical guide bar (50) between the excitation light source (13) and the irradiation plane.

5. The microorganism inspection apparatus (1) according to any one of claims 1 to 4, further comprising a shielding plate on a side opposite to the excitation light source across the irradiation plane.

## Patentansprüche

1. Vorrichtung zur Inspektion von Mikroorganismen (1) zum Messen der Anzahl von Mikroorganismen in einer Probenlösung, umfassend:
einen chargenartigen Probenbehälter, der aus einem lichtdurchlässigen Material gebildet ist;
Rühr-/Mischeinrichtung (7, 27) zum Rühren/Mischen einer Probe und eines fluoreszierenden Färbereagenz in dem chargenartigen Probenbehälter (5), um eine Probenlösung zu bilden;
eine Anregungslichtquelle (13), die eine Lichtquelle (10) aufweist, die eine horizontale Bestrahlungsebene des Probenbehälters (5) mit einem Anregungslichtstrahl in einer vertikalen Richtung bestrahlt, während die Probenlösung von der Rühr-/Mischeinrichtung gerührt wird;
Lichtempfangseinrichtung (14), die an einer seitlichen Stirnseite des Probenbehälters (5) angeordnet ist, wobei die Lichtempfangseinrichtung so konfiguriert ist, dass sie einen Fluoreszenzstrahl detektiert, der als Reaktion auf den Anregungslichtstrahl von der Anregungslichtquelle (13) emittiert wird; und
Steuerungseinrichtung (23) zum Umwandeln des von der Lichtempfangseinrichtung (14) detektierten Strahls in ein elektrisches Signal, um die Anzahl von Lichtemissionen zu detektieren, und Berechnen der Anzahl der in der Probe im Probenbehälter (5) enthaltenen Mikroorganismen aus der Anzahl von Lichtemissionen, wobei
die Rühr-/Mischeinrichtung (7, 27) und die Anregungslichtquelle (13) derart angeordnet sind, dass eine Richtung des Durchflusses der Probenlösung aufgrund der Bewegung durch die Rühr-/Mischeinrichtung und eine Richtung der Bestrahlung des Anregungslichtstrahls von der Anregungslichtquelle senkrecht zueinander sind.

2. Vorrichtung zur Inspektion von Mikroorganismen (1) nach Anspruch 1, wobei die Anregungslichtquelle (13) oberhalb oder unterhalb des Probenbehälters (5) angeordnet ist.

3. Vorrichtung zur Inspektion von Mikroorganismen (1) nach Anspruch 1,
wobei:
ein reflektierendes Element in dem Probenbehälter bereitgestellt ist,
die Anregungslichtquelle (13) an einer seitlichen Seite des Probenbehälters (5) angeordnet ist, und
ein von der Anregungslichtquelle emittierter Anregungslichtstrahl die Bestrahlungsebene in vertikaler Richtung bestrahlt, indem er von dem reflektierenden Element reflektiert wird.

4. Vorrichtung zur Inspektion von Mikroorganismen (1) nach einem der Ansprüche 1 bis 3, ferner umfassend eine optische Führungsschiene (50) zwischen der Anregungslichtquelle (13) und der Bestrahlungsebene.

5. Vorrichtung zur Inspektion von Mikroorganismen (1) nach einem der Ansprüche 1 bis 4, ferner umfassend eine Abschirmplatte auf einer Seite, die der Anregungslichtquelle über der Bestrahlungsebene gegenüberliegt.

## Revendications

1. Appareil d'inspection de micro-organismes (1) destiné à mesurer le nombre de micro-organismes dans une solution d'échantillon, comprenant :
un récipient d'échantillon de type lot formé d'un matériau transmettant la lumière ;
un moyen d'agitation/de mélange (7, 27) destiné à agiter/mélanger un échantillon et un réactif de coloration fluorescente dans le récipient d'échantillon (5) de type lot pour former une solution d'échantillon ;
une source de lumière d'excitation (13) comportant une source de lumière (10) qui irradie un plan d'irradiation horizontal du récipient d'échantillon (5) avec un faisceau de lumière d'excitation dans une direction verticale pendant que la solution d'échantillon est agitée par le moyen d'agitation/de mélange ;
un moyen de réception de lumière (14) disposé sur un côté de face latérale du récipient d'échantillon (5), le moyen de réception de lumière étant configuré pour détecter un faisceau de fluorescence émis en réponse au faisceau de lumière d'excitation provenant de la source de lumière d'excitation (13) ; et
un moyen de commande (23) destiné à convertir le faisceau détecté par le moyen de réception de lumière (14) en un signal électrique pour détecter le nombre d'émissions de lumière, et destiné à calculer le nombre de micro-organismes contenus dans l'échantillon dans le récipient d'échantillon (5) à partir du nombre d'émissions de lumière, dans lequel
le moyen d'agitation/de mélange (7, 27) et la source de lumière d'excitation (13) sont disposés de telle sorte qu'une direction de l'écoulement de la solution d'échantillon résultant de l'agitation par le moyen d'agitation/de mélange et une direction de l'irradiation du faisceau de lumière d'excitation provenant de la source de lumière d'excitation soient perpendiculaires l'une à l'autre.

2. Appareil d'inspection de micro-organismes (1) selon la revendication 1, dans lequel la source de lumière d'excitation (13) est disposée au-dessus ou au-dessous du récipient d'échantillon (5).

3. Appareil d'inspection de micro-organismes (1) selon la revendication 1,
dans lequel :
un élément réfléchissant est prévu dans le récipient d'échantillon,
la source de lumière d'excitation (13) est disposée sur un côté latéral du récipient d'échantillon (5), et
un faisceau lumineux d'excitation émis par la source lumineuse d'excitation irradie le plan d'irradiation dans la direction verticale en étant réfléchi par l'élément réfléchissant.

4. Appareil d'inspection de micro-organismes (1) selon l'une quelconque des revendications 1 à 3, comprenant également une barre de guidage optique (50) entre la source de lumière d'excitation (13) et le plan d'irradiation.

5. Appareil d'inspection de micro-organismes (1) selon l'une quelconque des revendications 1 à 4, comprenant également une plaque de blindage sur un côté opposé à la source de lumière d'excitation à travers le plan d'irradiation.
